# EUROPÄISCHE PATENTSCHRIFT

(11) **EP 3 860 674 B1**
(45) Veröffentlichungstag und Bekanntmachung des Hinweises auf die Patenterteilung: **21.09.2022**
(21) Anmeldenummer: 19706932.1
(22) Anmeldetag: 15.02.2019
(51) Int. Cl.: A61M 60/109, A61M 60/38, A61M 60/279, A61M 1/36

(54) **ARTERIELLE KANÜLE**
ARTERIAL CANNULA
CANULE ARTÉRIELLE

(43) Veröffentlichungstag der Anmeldung: 11.08.2021
(73) Patentinhaber: POLLARI, Francesco, 90480 Nürnberg (DE); Cuomo, Michela, 90480 Nürnberg (DE)
(72) Erfinder: POLLARI, Francesco, 90480 Nürnberg (DE); Cuomo, Michela, 90480 Nürnberg (DE)
(74) Vertreter: Stippl Patentanwälte
(86) Internationale Anmeldenummer: PCT/EP2019/053763
(87) Internationale Veröffentlichungsnummer: WO 2020/164727

(56) Entgegenhaltungen:
- EP-A2- 1 990 067
- WO-A1-97/37716
- WO-A1-2006/019714
- US-A- 5 584 803
- US-A1- 2002 016 566
- US-B1- 6 508 777

## Beschreibung

Die vorliegende Erfindung betrifft eine arterielle Kanüle für den Anschluss an eine Herz-Lungen-Maschine bzw. an ein Mechanical Circulatory Support System (MCS) zur Gewährleistung einer Blutzirkulation des gesamten Körpers zum Beispiel im Falle einer minimal-invasiven Herzoperation, hochriskanten perkutanen Koronareingriffen, bei Herzstillstand oder einem Low-Cardiac-Output-Syndrom.

### Technologischer Hintergrund

Ein Herz-Kreislauf-Bypass-System oder auch umgangssprachlich Herz-Lungen-Maschine genannt, wird eingesetzt, um die Funktionen des Herzens und der Lungen zeitweise zu ersetzen, indem ein Fluss von mit sauerstoffreichem Blut dem Kreislaufsystem des Patienten zugeführt wird. Hierbei wird sauerstoffarmes Blut aus dem Venensystem des Patienten abgeleitet, durch einen Blut-Oxygenator gepumpt und das sauerstoffreiche Blut anschließend zurück in das Arteriensystem des Patienten geführt, um eine arterielle Perfusion des Körpers zu ermöglichen.

Um eine zentrale Perfusion des Körpers mit sauerstoffreichem Blut im Vorwärtsfluss (Fluss vom Aorta ascendens zur absteigenden Aorta) zu erreichen, wird bisher eine vergleichsweise kurze Kanüle direkt am Aorta ascendens oder Bogen gesetzt. Diese Methode wird "zentrale Kannulation" genannt. Eine zentrale Kannulation erfordert eine Sternotomie (Längsdurchtrennung des Brustbeins) sowie Operation an der offenen Brust.

Es gibt jedoch bereit eine Methode, bei der sauerstoffreiches Blut über eine periphere Kanüle der Aorta zugeführt wird. Die Kanüle wird hierbei über die Oberschenkelarterie eingeführt. In diesem Fall ist der Blutstrom invert, da das sauerstoffreiche Blut von einem peripheren Gefäß (z.B. der arteria femoralis) zu der Aorta zurück fließen muss, um die lebenswichtigen Organe wie Gehirn, Nieren, Lungen sowie den Verdauungsorgane zu erreichen. Es handelt sich hierbei um einen minimalinvasiven Eingriff, welcher lediglich das Einführen der Kanüle in die Oberschenkelarterie voraussetzt. Sternotomie sowie Offenbrustoperation können vermieden werden. Allerdings besteht hierbei ein höheres Risiko einer zerebralen Schädigung durch eine Embolie, mobilisiert in der absteigenden Aorta durch den dort inverten Blutstrom. Zudem erhalten im Falle einer verbleibenden Restherzaktivität die supra-aortalen Blutgefäße mehr sauerstoffarmes Blut vom Herz im Vorwärtsstrom als sauerstoffreiches Blut über die Herz-Lungen-Maschine wegen des inverten Blutstroms.

Infolgedessen existiert ein gewisses Spannungsfeld zwischen der Anforderung eines möglichst patientenschonenden Eingriffs einerseits sowie dem Ausschluss von Komplikationen andererseits.

### Druckschriftlicher Stand der Technik

Eine arterielle Kanüle gemäß dem Oberbegriff des Anspruchs 1 ist aus der US 4,173,981 bekannt. Hierbei handelt es sich um eine im Beckenbereich einzusetzende arterielle Kanüle, die einen operativen Schnitt (Inzision) der Oberschenkelarterie erfordert und ohne eine Einführhilfe in Letztere eingeführt wird. Die Kanüle ist gerade ausgebildet und mit einer sich zum vorderen Ende verjüngenden Form ausgestattet. Des Weiteren weist sie jeweils maximal zwei, einseitig an der Kanüle angeordnete, kreisrund ausgebildete Perforationen an bestimmten Stellen entlang der Kanüle auf, an denen sauerstoffreiches Blut aus der Kanüle austreten kann. Die Öffnungen befinden sich an Orten von der Hauptarterie abzweigenden Arterien (wie z. B. supra-aortale Äste, Nierenarterie und Arteria iliaca). Neben der sich verjüngenden Form muss das Material der Kanüle einerseits belastbar andererseits flexibel sein, um ein Einsetzen und Manövrieren der Kanüle innerhalb der Aorta zu ermöglichen.

Aus der DE 699 11 950 T2 ist ein Kreislaufunterstützungssystem mit einem Arterien-Kanülen-Einführungs-Subsystem bekannt, bei dem im vorderen Endbereich der Kanüle ein Verschlussballon nach einer Mehrzahl von einzelnen einseitig an der Kanüle angebrachten Perforationen vorgesehen ist. Der Ballon wird nach dem Setzen der Kanüle durch einen zusätzlichen Kanal, der in der Kanüle wandseitig angeordnet ist, aufgepumpt. Zum Setzen der Kanüle kann ein Führungsdraht verwendet werden. Aufgrund des Verschlusses durch den Ballon kann kein sauerstoffreiches Blut über die Öffnungen auf die zum Ballon stromabseitige Seite gelangen. Daraus resultiert, dass der Aortabogen gerade im Bereich des Herzausgangs nicht mit Sauerstoff angereichertem Blut versorgt wird. Zudem besteht die Gefahr, dass der Ballon ein Blutgefäß (z. B. ein Gehirngefäß) versehentlich okkludieren kann. Weitere Beispiele von arteriellen Kanülen sind aus US2002016566 und US6,508,777 bekannt.

### Aufgabe der vorliegenden Erfindung

Die Aufgabe der vorliegenden Erfindung besteht darin, eine arterielle Kanüle zur Verfügung zu stellen, mit der bei Reduzierung der Komplikationsanfälligkeit eine gute Versorgung mit sauerstoffangereichertem Blut ermöglicht wird.

### Lösung der Aufgabe

Die vorliegende Aufgabe wird durch die Merkmale des Anspruchs 1 gelöst. Zweckmäßige Ausgestaltungen der Erfindung sind in den abhängigen Ansprüchen beansprucht.

Dadurch, dass der vordere Endbereich des rohrförmigen Körpers (vollständig oder zumindest teilweise) vorgekrümmt ist, eine Einführhilfe vorgesehen ist, die sich zum Setzen der Kanüle innerhalb rohrförmigen Körpers befindet und in den rohrförmigen Körper einschiebbar bzw. nach dem Setzen der Kanüle aus letzterem herausziehbar ist, so dass sich die Krümmung des vorderen Endbereichs des rohrförmigen Körpers nach dem Setzen der Kanüle und Herausziehen der Einführhilfe selbsttätig einstellt, wird erreicht, dass der vordere Bereich der Kanüle einerseits optimal in dem Aorta-Bogen positioniert und die Kanüle andererseits über die relativ lange Distanz von Oberschenkelarterie über Aorta bis hin zum Aorta-Bogen besonders Gefäß-schonend eingesetzt werden kann. Die Gefahr von Plaque-Ablösungen während des Setzens bzw. Einführens der Kanüle wird erheblich verringert. Ferner kann ein rohrförmiger Körper mit im Wesentlichen gleichbleibendem Durchmesser bzw. Lumen, zumindest was dessen vorderen Endbereich sowie Hauptbereich anbelangt, verwendet werden. Daraus wiederum resultiert, dass im Vergleich zu einbekannten er Kanüle mit sich nach vorne reduzierendem Durchmesser unter geringerem Druck eine ausreichende Volumenmenge an sauerstoffreichem Blut hin zum Aorta-Bogen transportiert werden und der gesamte Aorta-Bogen damit ausreichend versorgt werden kann. Die Kanüle wird durch eine perkutane Technik angelegt. Durch eine Punktion der Oberschenkelarterie wird ein Draht durch die Oberschenkelarterie bis zum Aorta-Bogen vorgeschoben. Auf den Draht kann dann die Kanüle zusammen mit der Einführhilfe (bzw. Dorn) bis in die Position in den Aorta-Bogen geschoben werden. Diese perkutane Methode ist als "Seldinger-Technik" bekannt und wird zur Anlage von Katheter und anderen Geräten im Körper ohne chirurgischen Aufschnitt verwendet. Die erfindungsgemäße Kanüle ermöglicht damit eine Zufuhr an sauerstoffreichem Blut wie dies bisher bei der zentralen Kannulation möglich war, jedoch ohne notwendigem operativen Eingriff und zwar weder direkt an dem Aorta-Bogen noch an der Oberschenkelarterie.

Dadurch, dass Perforationen ausschließlich im vorderen Endbereich des rohrförmigen Körpers, also im Gegensatz zum Stand der Technik nicht auch in dessen Hauptbereich, vorgesehen sind, wird die Gefahr von Komplikationen verringert. Denn im Hauptbereich vorgesehene Perforationen begründen die Gefahr, dass während des Einsatzes der Kanüle nicht erkannt wird, wenn im vorderen Bereich des rohrförmigen Körpers Perforationen (ggf. durch eine Lage der Kanüle mit Wandkontakt) verschlossen sind. Denn es erfolgt kein an der Druckmesseinrichtung wahrnehmbarer Druckanstieg, da sauerstoffreiches Blut über die im Hauptteil positionierten Perforierungen nach wie vor entweichen kann.

Erfindungsgemäß weist der rohrförmige Körper ein einziges Lumen auf. Zum einen ergibt sich hierdurch keine Versteifung des rohrförmigen Körpers in einer Vorzugsorientierung, die wiederum das Einführen der Kanüle erschweren würde, zum anderen ist die Kanüle auch sehr einfach technisch herstellbar.

Gemäß einer zweckmäßigen Ausgestaltung der erfindungsgemäßen Kanüle ist das Lumen kreisförmig ausgebildet.

Zweckmäßigerweise weisen das Lumen und/oder der rohrförmige Körper einen in Längsrichtung der Kanüle im vorderen Endbereich und/oder im Hauptbereich einen zumindest im Wesentlichen gleichbleibenden Durchmesser D1 bzw. D2 auf.

Dadurch, dass der vordere Endbereich des rohrförmigen Körpers nicht aufweitbar und/oder aufblasbar ist, kann sauerstoffangereichertes Blut über die Kanüle bis hin zum Herzausgang gelangen. Zudem müssen keine zusätzlichen Kanäle für das Zuführen von Druckgas zum Aufweiten der Kanüle vorgesehen sein.

Gemäß einer zweckmäßigen Ausgestaltung sind mindestens sechs, besonders vorzugsweise mindestens zwölf Perforierungen am vorderen Endbereich der Kanüle bzw. des rohrförmigen Körpers angeordnet. Hierdurch wird erreicht, dass eine ausreichende Menge an sauerstoffreichem Blut unter Vermeidung eines zu hohen Drucks im Aorta-Bogen zugeführt wird.

Dadurch, dass die Perforierungen, vorzugsweise symmetrisch, entlang des Umfangs des rohrförmigen Körpers herum angeordnet sind, wird erreicht, dass eine ausreichende Versorgung mit sauerstoffreichem Blut auch dann sichergestellt ist, wenn einige Perforierungen aufgrund eines Wandkontakts keinen ausreichenden Austritt von sauerstoffreichem Blut ermöglichen.

Dadurch, dass die Perforierungen eine Form mit einer Vorzugsorientierung, insbesondere eine langgestreckte und/oder ovale Form, besitzen, wird ein verbesserter Austritt von sauerstoffreichem Blut in Fließrichtung entlang der Wandung des rohrförmigen Körpers erzielt.

Dadurch, dass sich die Vorzugsorientierung der Form der Perforierungen in Längsrichtung des rohrförmigen Körpers erstreckt, kann vermieden werden, dass sich die Spitze der Einführhilfe versehentlich in einer Perforierung verfängt und/oder sich unter Umständen sogar durch eine Perforierung hindurch nach außen bewegt.

Alternativ können die Perforierungen auch einen runden Durchmesser aufweisen.

Dadurch, dass die Perforierungen einen Lochdurchmesser D3 bzw. Lochweite aufweisen, der bzw. die sich von der Innenseite zur Außenseite des rohrförmigen Körpers hin, vorzugsweise kontinuierlich, vergrößert, wird die Gefahr reduziert, dass durch die äußeren Kanten des rohrförmigen Körpers im Bereich der Perforierungen beim Einführen der Kanüle bzw. des vorderen Endbereichs des rohrförmigen Körpers Plaque Ablagerungen innerhalb der Aorta abgelöst werden.

Besonders zweckmäßig ist es, wenn die Perforierungen an der Außenseite des rohrförmigen Körpers abgerundet sind, d. h. von der Außenwand zur Innenwand des rohrförmigen Körpers abgerundet sind, sodass sich die Perforierungen in einem runden Verlauf von der Innenwand zur Außenwand öffnen, d. h. eine kontinuierlich verlaufende Aufweitung aufweisen.

Zur Überwachung des Einführens des rohrförmigen Körpers sind, zweckmäßigerweise am vorderen Endbereich desselben, Marker, insbesondere sogenannte Kontrastmittelmarker (z. B. in Form von röntgendichtem Material), vorgesehen.

Dadurch, dass die Marker den Bereich der Perforierungen und/oder den Bereich der Krümmung festlegen, kann die genaue Lage des jeweiligen Bereichs während des Einsetzens der Kanüle überwacht werden. Zweckmäßigerweise befindet sich ein Marker an dem distalen Ende des rohrförmigen Körpers. Ein weiterer Marker befindet sich zu Beginn des Abschnitts, der die Perforierungen enthält.

Gemäß einer weiteren Ausgestaltung kann der vordere Endbereich der Kanüle an seinem distalen Ende einen, zumindest im Wesentlichen, geraden Abschnitt aufweisen, in dem sich, vorzugsweise ausschließlich, die Perforierungen befinden. Der vorgekrümmte Bereich des vorderen Endbereichs der Kanüle kann ohne Perforierungen ausgestaltet sein. Dies kann zusätzliche Handhabungsvorteile mit sich bringen.

Zweckmäßigerweise weist die Einführhilfe einen Dorn (oder auch Straightener oder Strecker) auf, welcher in den rohrförmigen Körper einschiebbar ist und/oder durch diesen hindurchschiebbar ist, sodass die Kanüle zu Beginn des Einführens derselben in die Oberschenkelarterie sowie auch während des Hinführens von der Oberschenkelarterie über die Aorta bis hin zum Aorta-Bogen unter Überwindung der Vorkrümmung des vorderen Teils des rohrförmigen Körpers gestreckt ist. Neben der Streckung der Vorkrümmung der Kanüle dient die Einführhilfe bzw. der Dorn auch dazu, die nötige Starrheit zu gewährleisten, um die Kanüle vorzuschieben (ohne dass sich diese biegt). Der rohrförmige Körper sowie der Dorn können hierbei von dem ausführenden Arzt unabhängig voneinander verschoben werden. Vorzugsweise weist der Dorn an seinem distalen Ende eine Rundung und/oder eine allmähliche zum distalen Ende hin verlaufende Querschnittsverjüngung auf.

Die Einführhilfe umfasst einen äußerst flexiblen Draht, welcher in den rohrförmigen Körper einschiebbar und/oder durch diesen hindurchschiebbar ist und mittels dem die Richtung des vorderen Endbereichs des rohrförmigen Körpers, vorzugsweise des rohrförmigen Körpers sowie des Dorns, beim Setzen der Kanüle durch arterienwandberührungsbedingte Krümmung des Drahtes zusätzlich beeinflussbar ist. Der Draht wird vorgelegt, damit die Kanüle und die Einführhilfe bzw. der Dorn in die durch den Draht festgelegte Richtung vorangeschoben werden können. Die Einführhilfe bzw. der Dorn umfasst hierzu einen Kanal, in dem der Draht verläuft.

Sowohl der Dorn als auch der Draht werden nach dem Setzen der Kanüle entfernt.

Gemäß einer weiteren Ausgestaltung kann die Kanüle, vorzugsweise in deren rückseitigen Endbereich, mit einer Abzweigung sowie einem Hahn ausgestattet sein. Hierdurch kann das System bei Bedarf entlüftet und/oder gespült werden bzw. an eine Druckmesseinrichtung angeschlossen werden.

### Beschreibung der Erfindung anhand von Ausführungsbeispielen

Zweckmäßige Ausgestaltungen der vorliegenden Erfindung werden nachstehend anhand von Zeichnungsfiguren näher erläutert. Es zeigen:
- Fig. 1: eine Übersichtsdarstellung der Ausgestaltung einer erfinderischen arteriellen Kanüle im eingesetzten Zustand;
- Fig. 2: eine Darstellung eines Beispiels einer erfinderischen Kanüle im Ausgangszustand in Draufsicht (Fig. 2A) sowie im Schnitt entlang der Schnittlinie I-I (Fig. 2B);
- Fig. 3: die Kanüle gemäß Fig. 2 mit eingesetzter Einführhilfe in Seitenansicht (Fig. 3A), im Schnitt entlang der Schnittlinie I-I (Fig. 3B) sowie in Draufsicht mit einer teilweise zurückgezogenen Einführhilfe (Fig. 3C);
- Fig. 4: die Lagepositionierung des vorderen Teils einer Ausgestaltung der erfindungsgemäßen Kanüle nach vollendeter Lagepositionierung jedoch noch mit Einführhilfe;
- Fig. 5: eine Schnittdarstellung des vorderen Teils des rohrförmigen Körpers mit einer ersten Ausgestaltung der Form der Perforationen (Fig. 5A) sowie einer zweiten Ausgestaltung der Form der Perforationen (Fig. 5B),
- Fig. 6: eine Darstellung eines weiteren Beispiels eines Teils einer erfindungsgemäßen Kanüle in Draufsicht sowie
- Fig. 7: eine Darstellung eines weiteren Beispiels einer erfinderischen Kanüle im Ausgangszustand in Draufsicht (Fig. 7A) sowie im Schnitt entlang der Schnittlinie I-I (Fig. 7B);.

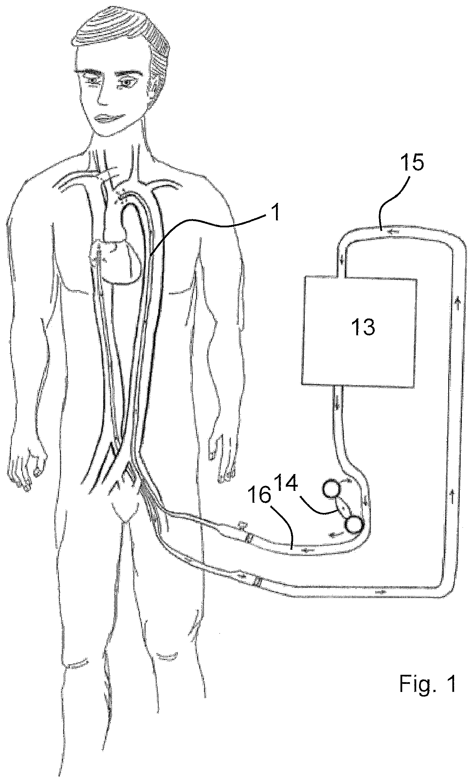

Fig. 1 zeigt schematisch den Anschluss einer sogenannten Herz-Lungen-Maschine bzw. eines sogenannten Mechanical Circulatory Support Systems (MCS) zur Gewährleistung einer Blutzirkulation des gesamten menschlichen Körpers beispielsweise im Falle einer Herzoperation oder dergleichen. Die Lungen-Maschine umfasst einen sogenannten Oxygenator 13, in dem Blut mit Sauerstoff angereichert wird. Das Blut des Patienten wird hierzu aus dessen Venensystem über ein geeignetes Zufuhrschlauchsystem 15 abgeleitet und dem Oxygenator 13 zugeführt. Von dort wird es nach Sauerstoffanreicherung über eine Pumpe 14 sowie ein geeignetes Abführschlauchsystem 16 dem Arteriensystem des Patienten wieder zugeführt. Hierzu wird erfindungsgemäß eine Kanüle 1, verwendet, die im Bereich der Oberschenkelarterie eingesetzt wird und von der Oberschenkelarterie über die Aorta bis hin zum Aorta-Bogen verläuft. Die Kanüle 1 wird unter Anwendung der sogenannten Seldinger-Methode gesetzt. Das bedeutet, es wird keine Inzision (operativer Schnitt) der Oberschenkelarterie im Bereich des Eintritts der Kanüle 1 vorgenommen. Mittels der erfindungsgemäßen Kanüle 1 wird sauerstoffreiches Blut vom Oxygenator über die Kanüle direkt in den Bereich des Aorta-Bogens gebracht, um eine zentrale Perfusion des Körpers mit sauerstoffreichem Blut im Vorwärtsfluss (Fluss vom Aorta-Bogen zur absteigenden Aorta) zu erreichen. Im Gegensatz zu einer zentralen Kannulation wird somit keine Sternotomie (Längsdurchtrennung des Brustbeins) sowie Operation an der offenen Brust notwendig.

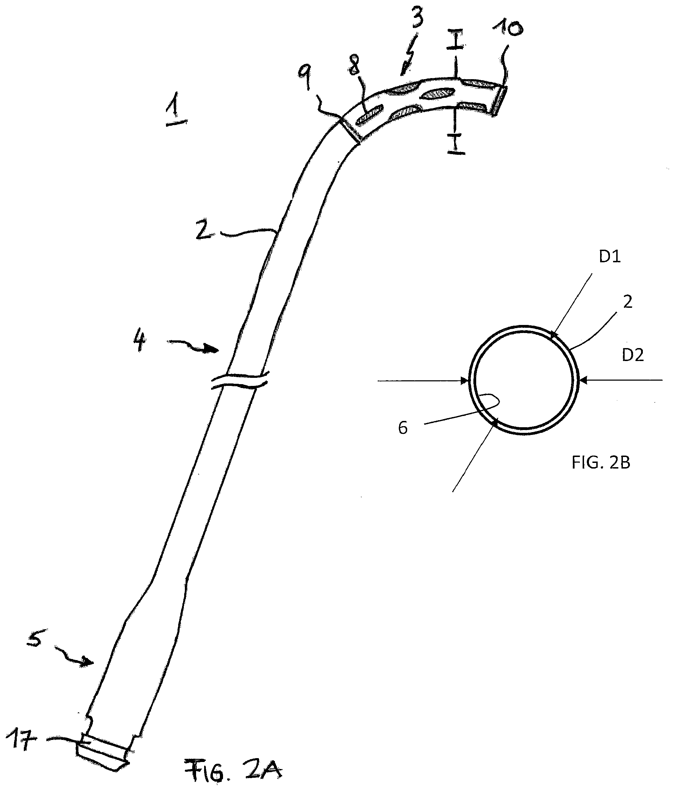

Fig. 2A zeigt eine erste Ausgestaltung der erfindungsgemäßen Kanüle 1 in einer Draufsicht. Die Kanüle 1 umfasst einen rohrförmigen Körper 2 mit einem vorderen (distalen) Endbereich 3, einem Hauptbereich 4 sowie einem unteren d. h. proximalen Endbereich 5 mit einem Anschlussstutzen 17, der dazu dient, das Abführschlauchsystem 16 des Oxygenators 13 anzuschließen.

Der rohrförmige Körper 2 besitzt, vgl. Fig. 2B, ein einziges Lumen 6, welches kreisförmig ist und einen Durchmesser D1 aufweist. Der rohrförmige Körper 2 ist ebenfalls kreisförmig und besitzt einen Durchmesser D2. Der Durchmesser D1 liegt in einem Bereich von 16 bis 18 Fr. Im unteren Endbereich beträgt der Durchmesser des Lumens 28 bis 34 Fr.

Zweckmäßigerweise kann der rohrförmige Körper 2 im Hauptbereich 4 und/oder im vorderen Endbereich 3 einen gleichbleibenden Durchmesser D1 und/oder D2 aufweisen.

Im vorderen Endbereich besitzt der rohrförmige Körper 2 erfindungsgemäß eine Vorkrümmung. Im Bereich dieser Vorkrümmung sind eine Mehrzahl von Perforierungen 8 vorgesehen, die vorzugsweise entlang des Umfangs des rohrförmigen Körpers 2 positioniert sind, sodass bei einem ungewollten Verschluss einer Perforierung oder eines Teils der Perforierungen z. B. durch Gefäßwandanlage die übrigen Perforierungen 8 frei bleiben. Die Perforierungen 8 weisen bei der in Fig. 2A gezeigten Variante eine längliche Vorzugsorientierung auf, die z. B. in Längsrichtung der Kanüle gerichtet sein können. Zudem können die Perforierungen in symmetrischer Anordnung zueinander entlang des Umfangs des rohrförmigen Körpers 2 angeordnet sein.

Das distale Ende des rohrförmigen Körpers 2 ist offen.

Die Kanüle 1 bzw. deren rohrförmiger Körper 2 weisen eine ausreichende Länge auf, damit die Kanüle 1 von der Oberschenkelarterie bis in den Aorta-Bogen reicht. Die Kanüle 1 bzw. der rohrförmige Körper 2 können dementsprechend eine Länge von ca. 40 bis 60 cm aufweisen.

Die Kanüle 1 bzw. der rohrförmige Körper 2 können mit einem die Blutgerinnung verhindernden Material beschichtet sein, beispielsweise in Form einer sogenannten Heparin-haltigen Beschichtung. Der rohrförmige Körper 2 besteht aus Kunststoff oder einem Material auf Kunststoffbasis, welches flexibel ist.

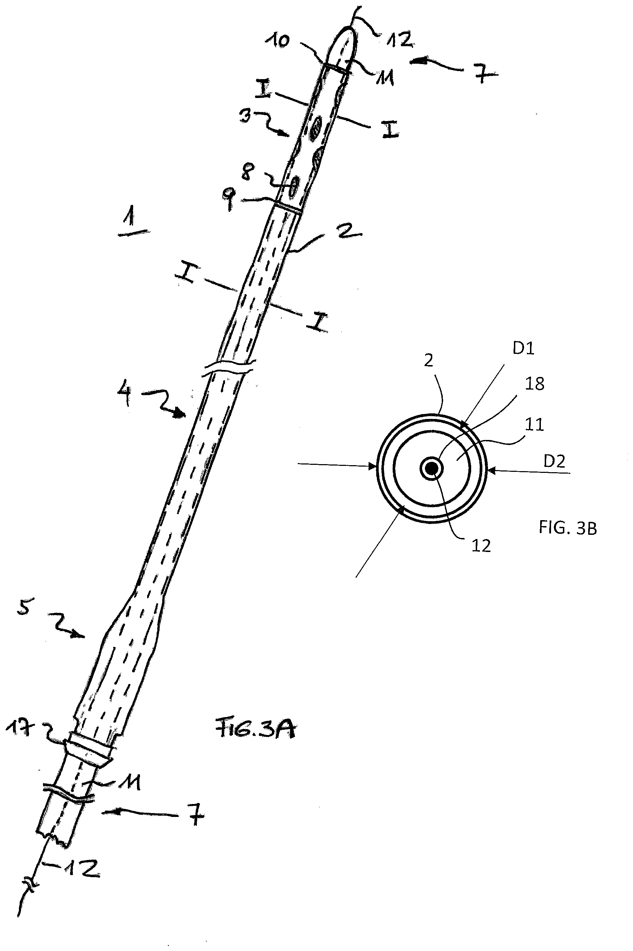

Der vordere Endbereich 3 des rohrförmigen Körpers ist im Ausgangszustand vorgekrümmt. Die Vorkrümmung ist flexibel und dergestalt, dass sie mittels einer Einführhilfe (vgl. Fig. 3A) durch Streckung des rohrförmigen Körpers 2 aufgehoben werden kann. Die Kanüle 1 wird wie in der in Fig. 2A gezeigten Form zur Anwendung bereitgehalten.

Im vorderseitigen Endbereich 3 ist jeweils ein Kontrastmittelmarker 9 zu Beginn des Bereichs der Perforierungen 8 sowie am distalen Ende des rohrförmigen Körpers 2 vorgesehen. Der jeweilige Kontrastmittelmarker 9, 10 dient dazu, die Position der Kanüle bzw. des vorderseitigen Endbereichs 3 während des Einsetzens indirekt also über die Position der Kontrastmittelmarker 9, 10 zu überprüfen.

Erfindungsgemäß besitzt die Kanüle im vorderen Endbereich 3 keinen erweiterbaren oder aufblasbaren Bereich. Darüber hinaus sind Perforierungen lediglich im vorderen Endbereich 3, nicht jedoch im Hauptbereich 4 vorgesehen.

Zum Einführen der Kanüle ist eine Einführhilfe 7 vorgesehen, mittels welcher die Vorkrümmung des vorderen Endbereichs 3 des rohrförmigen Körpers 2 aufgehoben d. h. der rohrförmige Körper 2 begradigt werden kann. Hierzu umfasst die Einführhilfe 7 einen Dorn 11 oder auch Straightener genannt, welcher innerhalb des rohrförmigen Körpers 2 verschiebbar angeordnet ist und an seinem vorderen distalen Ende abgerundet ist. Der Dorn 11 wird im Endbereich 5 an dem Stutzen 17 entweder zentral herausgeführt, wie in Fig. 3A dargestellt, oder es ist eine zusätzliche (in Fig. 3A nicht dargestellte) Abzweigung für den Dorn 11 vorgesehen. Der Dorn 11 kann zweckmäßigerweise ebenfalls aus einem Kunststoffmaterial bestehen, welches eine Steifigkeit aufweist, die größer ist als die Rückstellkraft der Krümmung im oberen Bereich 3 des rohrförmigen Körpers 2.

Darüber hinaus umfasst die Einführhilfe 7 zusätzlich einen flexiblen Draht 12, welcher zum Einschieben des Dorns 11 sowie des rohrförmigen Körpers 2 dient. Der Draht 12 verläuft beispielsweise in einem im Dorn 11 vorgesehenen Führungskanal 18, der den gesamten Dorn 11 durchsetzt, sodass der Draht an der Vorderseite des distalen Endes des Dorns 11 herausragt. Beim Setzen der Kanüle 1 wird zunächst der Draht 12 bis zum Aorta-Bogen eingeführt. Durch einen Wandkontakt mit der Gefäßwand beim Vorwärtsschieben der Kanüle krümmt sich der Draht 12 entsprechend des Verlaufs der Blutgefäße. Hierdurch wird die Einschubrichtung der Kanüle 1 bzw. des Dorns 11 festgelegt.

Alternativ zu der in Fig. 3A dargestellten Ausgestaltung kann der vordere Bereich des Drahts 12 ebenfalls, beispielsweise durch Vorspannung desselben, eine Krümmung aufweisen. Die Krümmung kann z. B. so klein sein, dass sie durch den rohrförmigen Körper 2 beim Herausziehen nicht stört.

Die mit der Einführhilfe 7 ausgestattete Kanüle 1 kann aufgrund der Flexibilität des rohrförmigen Körpers 2 sowie des Dorns 11 über den vorab gelegten, d. h. eingeführten, Draht 12 entlang der Verwindungen der Oberschenkelarterie gut in die Aorta eingeschoben werden. Bei dem Draht 12 handelt es sich vorzugsweise um einen sehr engschraubig gewundenen Draht, vorzugsweise Stahldraht, der aufgrund seiner Windungen äußerst flexibel ist. Der Draht 12 kann zudem zur besseren Handhabung mit einem Kunststoffschlauch überzogen sein (was in Fig. 3A nicht dargestellt ist).

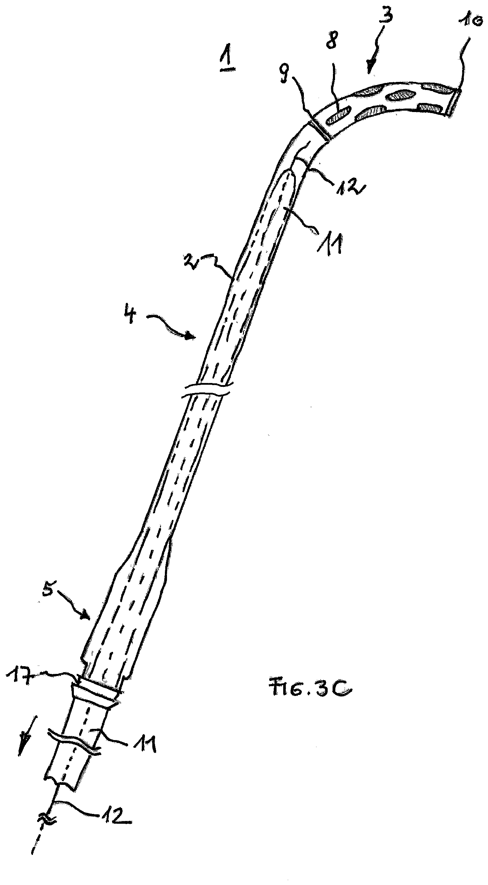

Nachdem der Katheder 1 in Position gebracht worden ist und der vordere Endbereich 3 des rohrförmigen Körpers 2 sich im Bereich des Aorta-Bogens befindet, wird die Einführhilfe 7 unter Beibehaltung der Position der Kanüle 1 behutsam aus letzterer herauszogen, wie dies in Fig. 3C dargestellt ist. Hierbei krümmt sich der vordere Endbereich mit zunehmender proximalseitiger Entfernung des Dorns 11 allmählich in seine vorher festgelegte Krümmung zurück. Anschließend wird der Dorn 11 zusammen mit dem Draht 12 aus der Kanüle 1 herausgezogen und der Endbereich 5 mit dem dort befindlichen Stutzen 17 mit dem Abführschlauchsystem 16 des Oxygenators 13 verbunden.

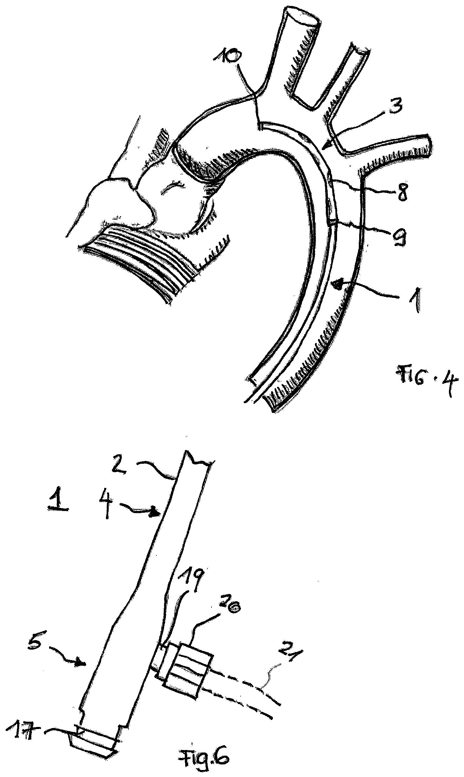

Fig. 4 zeigt den vorderen Endbereich 3 der Kanüle in dem im Aorta-Bogen korrekt eingesetzten Zustand. Aus der Darstellung wird deutlich, dass aufgrund der Vielzahl der in diesem Bereich vorgesehenen Perforierungen 8 und der Tatsache, dass der vordere Endbereich 3 der Kanüle keinen aufweitbaren bzw. aufblasbaren Bereich aufweist, sauerstoffreiches Blut in alle Bereiche des Aorta-Bogens sowie auch zum Herzausgang gelangen kann.

Die Perforierungen 8 sind vorzugsweise entlang des Umfangs des vorderseitigen Endbereichs 3 herum, also nicht nur einseitig, angeordnet. Des Weiteren können sie in einem bestimmten Anordnungsmuster, vorzugsweise in einem symmetrischen Anordnungsmuster, um den Umfang des rohrförmigen Körpers 2 herum angeordnet sein. Um eine ausreichende Versorgung mit sauerstoffreichem Blut bei vergleichsweise normalem also nicht zu hohem Druck zu gewährleisten, sind im vorderen Bereich 3 mindestens 4, vorzugsweise mindestens 6, besonders vorzugsweise mindestens 8 Perforierungen 8 vorgesehen. Beispielsweise können insgesamt 10 Perforierungen vorgesehen sein.

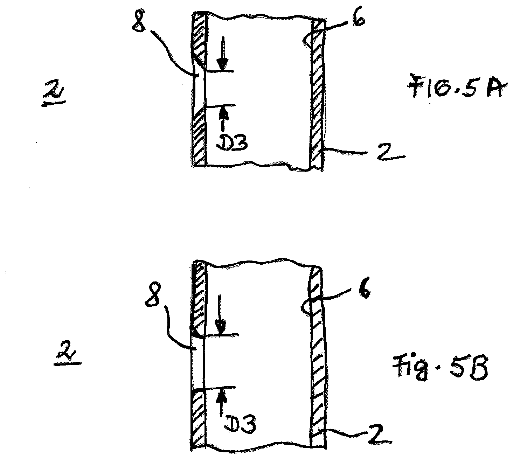

Gemäß zweckmäßigen in Fig. 5A und Fig. 5B gezeigten Ausgestaltungen der vorliegenden Erfindung sind die Perforierungen 8 derart ausgestaltet, dass sie eine Lochweite bzw. einen Lochdurchmesser D3 aufweisen, der sich von der Innenseite zur Außenseite des rohrförmigen Körpers 2 hin, vorzugsweise kontinuierlich, vergrößert, wie dies in Fig. 5A und Fig. 5B dargestellt ist. Dies hat den Vorteil, dass im Außenbereich der jeweiligen Perforierung 8 keine abrasive Kante existiert, durch die Ablagerungen wie z. B. Plaque an der Innenseite der Oberschenkelarterie oder Aorta versehentlich abgelöst werden.

Bei der Lochausgestaltung der Perforierung 8 gemäß Fig. 5A zeigt die Innenwand des Lochs eine sich gleichbleibend erweiternde Form.

Bei der Ausgestaltung nach Fig. 5B sind die Perforierungen 8 an der Außenseite des rohrförmigen Körpers 2 zur Innenseite hin abgerundet, was ebenfalls zu dem vorgenannten Effekt beiträgt.

Gemäß einer weiteren Ausgestaltung gemäß Fig. 6 kann die erfindungsgemäße Kanüle vorzugsweise in ihrem rückseitigen Endbereich 5 eine Abzweigung 19 aufweisen, an der ein Hahn 20 vorgesehen ist. Über den Hahn kann bei Bedarf über einen Zuführungsschlauch 21 nachgespült werden und/oder das System entlüftet werden. Ferner ermöglicht es die Abzweigung 19 bzw. der Hahn 20, das System mit einer (in den Zeichnungen nicht dargestellten) Druckmesseinrichtung zu verbinden.

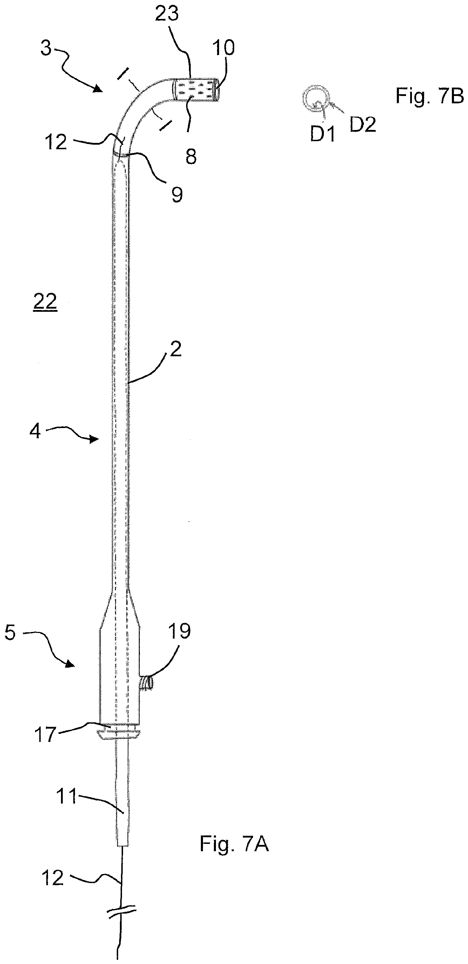

Fig. 7A zeigt eine weitere zweckmäßige Ausgestaltung der erfindungsgemäßen Kanüle 22, bei der der vordere Endbereich 3 des röhrenförmigen Körpers 2 in zwei Abschnitte unterteilt ist. Der eine Abschnitt ist mit der Vorkrümmung versehen und bildet im fertigen Gebrauchszustand die Krümmung aus, wohingegen der weitere Abschnitt 23 des vorderseitigen Endbereichs 3, zumindest im Wesentlichen, geradlinig ausgebildet ist. Bei der in Fig. 7A gezeigten Ausgestaltung befinden sich die Perforierungen 8 in dem weiteren Abschnitt 23. Ferner kann bei dieser Ausgestaltung der gekrümmte Bereich, d.h. der Bereich der Vorkrümmung ohne Perforierung ausgestaltet sein. Im Übrigen entspricht diese Ausgestaltung den bereits vorbeschriebenen Ausgestaltungen der erfindungsgemäßen Kanüle 1 mit all deren Variationen.

Zusammenfassend ist festzustellen, dass die vorliegende Erfindung eine neuartige arterielle Kanüle betrifft, die eine optimale Versorgung des Aorta-Bogens mit sauerstoffreichem Blut ohne Sternotomie sowie Operation an der offenen Brust ermöglicht. Dies ermöglicht eine ganz wesentliche Entlastung des Patienten. Zudem werden auch die Behandlungskosten und Regenerationskosten im Vergleich zu einer zentralen Kannulation erheblich reduziert. Im Übrigen kann die erfindungsgemäße Kanüle in einfacher und kostengünstiger Weise produziert werden.

### BEZUGSZEICHENLISTE

- 1: arterielle Kanüle
- 2: rohrförmiger Körper
- 3: vorderseitiger Endbereich
- 4: Hauptbereich
- 5: rückseitiger Endbereich
- 6: Lumen
- 7: Einführhilfe
- 8: Perforierung
- 9: Kontrastmittelmarker
- 10: Kontrastmittelmarker
- 11: Dorn
- 12: Draht
- 13: Oxygenator
- 14: Pumpe
- 15: Zufuhrschlauchsystem
- 16: Abführschlauchsystem
- 17: Stutzen
- 18: Führungskanal
- 19: Abzweigung
- 20: Hahn
- 21: Zuführungsschlauch
- 22: arterielle Kanüle
- 23: gerader Abschnitt

## Patentansprüche

1. Arterielle Kanüle (1, 22) für den Anschluss an eine Herz-Lungenmaschine zur Versorgung eines menschlichen Patienten mit sauerstoffreichem Blut umfassend
einen rohrförmigen Körper (2) mit einem vorderen Endbereich (3) zur Positionierung im Bereich des Aorta-Bogens des Patienten, einem Hauptbereich (4) sowie einem rückseitigen Endbereich (5) zum versorgungsseitigen Anschluss,
wobei der rohrförmige Körper (2) ein Lumen (6) aufweist,
wobei der rohrförmigen Körper (2) aus einem flexiblen Material besteht,
wobei die Länge des rohrförmigen Körper (2) derart dimensioniert ist, dass die Kanüle (1) im Bereich der Oberschenkelarterie des Patienten gelegt werden kann und sich bis in den Bereich des Aorta-Bogens erstreckt,
wobei der rohrförmigen Körper (2) dazu dient, einen Versorgungsstrom an sauerstoffreichem Blut hin zum Aorta-Bogen zu gewährleisten,
und wobei im vorderen Endbereich (3) des rohrförmigen Körper (2) Perforierungen (8) vorgesehen sind,
der vordere Endbereich (3) des rohrförmigen Körpers (2) in Anlehnung an die Form des Aorta-Bogens zumindest teilweise vorgekrümmt ist,
eine Einführhilfe (7) vorgesehen ist, die sich zum Setzen der Kanüle (1) innerhalb rohrförmigen Körpers (2) befindet und in den rohrförmigen Körper (2) einschiebbar bzw. nach dem Setzen der Kanüle (1) aus letzterem herausziehbar ist,
**dadurch gekennzeichnet, dass**
die Krümmung des vorderen Endbereichs (3) des rohrförmigen Körpers (2) sich nach dem Setzen der Kanüle (1, 22) und Herausziehen der Einführhilfe (7) selbsttätig einstellt, und
der rohrförmige Körper (2) ein einziges Lumen (6) aufweist.

2. Kanüle nach Anspruch 1, **dadurch gekennzeichnet, dass** Perforierungen (8) ausschließlich im vorderen Endbereich (3) des rohrförmigen Körpers vorgesehen sind.

3. Kanüle nach Anspruch 1 oder 2, **dadurch gekennzeichnet, dass** das Lumen (6) kreisförmig ist.

4. Kanüle nach den vorhergehenden Ansprüchen, **dadurch gekennzeichnet, dass** das Lumen (6) und/oder der rohrförmige Körper (2) einen in Längsrichtung der Kanüle (1, 22) im vorderen Endbereich (3) und/oder im Hauptbereich (4) einen zumindest im Wesentlichen gleichbleibenden Durchmesser D1 bzw. D2 aufweist bzw. aufweisen.

5. Kanüle nach den vorhergehenden Ansprüchen, **dadurch gekennzeichnet, dass** der vordere Endbereich (3) des rohrförmigen Körpers (2) nicht aufweitbar und/oder aufblasbar ist.

6. Kanüle nach den vorhergehenden Ansprüchen, **dadurch gekennzeichnet, dass** mindestens vier vorzugsweise mindestens sechs, besonders vorzugsweise mindestens acht Perforierungen (8) vorgesehen sind.

7. Kanüle nach den vorhergehenden Ansprüchen, **dadurch gekennzeichnet, dass** die Perforierungen (8), vorzugsweise symmetrisch, entlang des Umfangs des rohrförmigen Körpers (2) herum angeordnet sind.

8. Kanüle nach den vorhergehenden Ansprüchen, **dadurch gekennzeichnet, dass** die Perforierungen (8) eine Form mit Vorzugsorientierung, insbesondere eine langgestreckte und/oder ovale Form, besitzen.

9. Kanüle nach Anspruch 8, **dadurch gekennzeichnet, dass** die Vorzugsorientierung der Form der Perforierungen (8) sich in Längsrichtung des rohrförmigen Körpers (2) erstreckt.

10. Kanüle nach den vorhergehenden Ansprüchen, **dadurch gekennzeichnet, dass** die Perforierungen (8) einen Lochdurchmesser D3 aufweisen, der sich von der Innenseite zur Außenseite des rohrförmigen Körpers (2) hin, vorzugsweise kontinuierlich, vergrößert.

11. Kanüle nach den vorhergehenden Ansprüchen, **dadurch gekennzeichnet, dass** die Perforierungen (8) an der Außenseite des rohrförmigen Körpers (2) abgerundet sind.

12. Kanüle nach den vorhergehenden Ansprüchen, **dadurch gekennzeichnet, dass** am vorderen Endbereich (3) des rohrförmigen Körpers (2) Kontrastmittelmarker (9, 10) vorgesehen sind.

13. Kanüle nach Anspruch 12, **dadurch gekennzeichnet, dass** die Kontrastmittelmarker (9, 10) den Bereich der Perforierungen (8) und/oder den Bereich der Krümmung festlegen.

14. Kanüle nach Anspruch 12 oder 13, **dadurch gekennzeichnet, dass** ein Kontrastmittelmarker (9) am Beginn des Bereichs der Perforierungen (8) und ein Kontrastmittelmarker (10) am Ende des Bereichs der Perforierungen (8) oder am Ende des rohrförmigen Körpers (2) positioniert ist.

15. Kanüle nach den vorhergehenden Ansprüchen, **dadurch gekennzeichnet, dass** der vordere Endbereich (3) der Kanüle (22) an seinem distalen Ende einen, zumindest im Wesentlichen, geraden Abschnitt (23) aufweist, in dem sich, vorzugsweise ausschließlich, die Perforierungen (8) befinden.

16. Kanüle nach den vorhergehenden Ansprüchen, **dadurch gekennzeichnet, dass** die Einführhilfe (7) einen Dorn (11) aufweist, welcher in den rohrförmigen Körper (2) einschiebbar und/oder durch diesen hindurchschiebbar ist.

17. Kanüle nach den vorhergehenden Ansprüchen, **dadurch gekennzeichnet, dass** die Einführhilfe (7) einen Draht (12) umfasst.

18. Kanüle nach Anspruch 16 oder 17, **dadurch gekennzeichnet, dass** der Draht (12) im oder am Dorn (11) geführt ist.

## Claims

1. Arterial cannula (1, 22) for the connection to a heart-lung machine for supplying a human patient with oxygen-rich blood, comprising
a tubular body (2) having a front end region (3) for positioning in the region of the aortic arch of the patient, a main region (4), and a rear end region (5) for the supply-side connection,
wherein the tubular body (2) has a lumen (6), wherein the tubular body (2) is made of a flexible material,
wherein the length of the tubular body (2) is dimensioned such that the cannula (1) can be placed in the region of the femoral artery of the patient and extends into the region of the aortic arch,
wherein the tubular body (2) serves to ensure a supply flow of oxygen-rich blood to the aortic arch,
and wherein perforations (8) are provided in the front end region (3) of the tubular body (2),
the front end region (3) of the tubular body (2) is at least partially pre-curved following the shape of the aortic arch,
an insertion aid (7) is provided which, for placement of the cannula (1), is located inside the tubular body (2) and which is slidable into the tubular body (2) or, after placement of the cannula (1), removable from the tubular body (2),
**characterized in that**
the curvature of the front end region (3) of the tubular body (2) adjusts automatically after placement of the cannula (1, 22) and removal of the insertion aid (7), and
the tubular body (2) has a single lumen (6).

2. Cannula according to Claim 1, **characterized in that** perforations (8) are provided exclusively in the front end region (3) of the tubular body.

3. Cannula according to Claim 1 or 2, **characterized in that** the lumen (6) is circular.

4. Cannula according to the preceding claims, **characterized in that** the lumen (6) and/or the tubular body (2) have/has an at least substantially constant diameter D1, D2, respectively, in the longitudinal direction of the cannula (1, 22) in the front end region (3) and/or in the main region (4).

5. Cannula according to the preceding claims, **characterized in that** the front end region (3) of the tubular body (2) is non-expandible and/or non-inflatable.

6. Cannula according to the preceding claims, **characterized in that** at least four, preferably at least six, particularly preferably at least eight perforations (8) are provided.

7. Cannula according to the preceding claims, **characterized in that** the perforations (8) are arranged, preferably symmetrically, about the circumference of the tubular body (2).

8. Cannula according to the preceding claims, **characterized in that** the perforations (8) have a shape with a preferred orientation, in particular an elongate and/or oval shape.

9. Cannula according to Claim 8, **characterized in that** the preferred orientation of the shape of the perforations (8) extends in the longitudinal direction of the tubular body (2).

10. Cannula according to the preceding claims, **characterized in that** the perforations (8) have a hole diameter D3 which increases in size, preferably continuously, from the inside to the outside of the tubular body (2).

11. Cannula according to the preceding claims, **characterized in that** the perforations (8) are rounded off at an outer side of the tubular body (2).

12. Cannula according to the preceding claims, **characterized in that** contrast agent markers (9, 10) are provided at the front end region (3) of the tubular body (2) .

13. Cannula according to Claim 12, **characterized in that** the contrast agent markers (9, 10) define the region of the perforations (8) and/or the region of the curvature.

14. Cannula according to Claim 12 or 13, **characterized in that** a contrast agent marker (9) is positioned at the start of the region of the perforations (8), and a contrast agent marker (10) is positioned at the end of the region of the perforations (8) or at the end of the tubular body (2).

15. Cannula according to the preceding claims, **characterized in that** the front end region (3) of the cannula (22) has, at its distal end, an at least substantially straight portion (23) in which the perforations are preferably exclusively located.

16. Cannula according to the preceding claims, **characterized in that** the insertion aid (7) has a mandrel (11) which is slidable into the tubular body (2) and/or slidable through the latter.

17. Cannula according to the preceding claims, **characterized in that** the insertion aid (7) comprises a wire (12).

18. Cannula according to Claim 16 or 17, **characterized in that** the wire (12) is guided in or on the mandrel (11) .

## Revendications

1. Canule artérielle (1, 22) destinée à être raccordée à une machine cœur-poumon pour alimenter un patient humain en sang riche en oxygène, ladite canule comprenant un corps tubulaire (2) pourvu d'une zone d'extrémité avant (3) destinée à être positionnée au niveau de la crosse aortique du patient, une zone principale (4) et une zone d'extrémité arrière (5) destinée à être raccordée du côté alimentation,
le corps tubulaire (2) comportant une lumière (6),
le corps tubulaire (2) étant en une matière souple,
la longueur du corps tubulaire (2) étant dimensionnée de telle sorte que la canule (1) puisse être placée au niveau de l'artère fémorale du patient et s'étende jusqu'au niveau de la crosse aortique,
le corps tubulaire (2) servant à assurer un flux d'alimentation en sang riche en oxygène en direction de la crosse aortique,
et des perforations (8) étant ménagées dans la zone d'extrémité avant (3) du corps tubulaire (2),
la zone d'extrémité avant (3) du corps tubulaire (2) étant au moins partiellement précourbée en fonction de la forme de la crosse aortique,
un auxiliaire d'insertion (7) étant prévu qui est situé à l'intérieur du corps tubulaire (2) afin de placer la canule (1) et pouvant être poussé dans le corps tubulaire (2) ou retiré de celui-ci une fois que la canule (1) a été placée,
**caractérisée en ce que**
la courbure de la zone d'extrémité avant (3) du corps tubulaire (2) se règle automatiquement une fois que la canule (1, 22) a été placée et l'auxiliaire d'insertion (7) retiré, et
le corps tubulaire (2) comporte une seule lumière (6).

2. Canule selon la revendication 1, **caractérisée en ce que** des perforations (8) sont ménagées exclusivement dans la zone d'extrémité avant (3) du corps tubulaire.

3. Canule selon la revendication 1 ou 2, **caractérisée en ce que** la lumière (6) est circulaire.

4. Canule selon les revendications précédentes, **caractérisée en ce que** la lumière (6) et/ou le corps tubulaire (2) ont un diamètre D1 ou D2 au moins sensiblement constant dans la direction longitudinale de la canule (1, 22) dans la zone d'extrémité avant (3) et/ou dans la zone principale (4).

5. Canule selon les revendications précédentes, **caractérisée en ce que** la zone d'extrémité avant (3) du corps tubulaire (2) n'est pas extensible et/ou gonflable.

6. Canule selon les revendications précédentes, **caractérisée en ce qu'**au moins quatre, de préférence au moins six, de manière particulièrement préférée au moins huit, perforations (8) sont ménagées.

7. Canule selon les revendications précédentes, **caractérisée en ce que** les perforations (8) sont ménagées, de préférence symétriquement, sur toute la circonférence du corps tubulaire (2).

8. Canule selon les revendications précédentes, **caractérisée en ce que** les perforations (8) ont une forme présentant une orientation privilégiée, notamment une forme allongée et/ou ovale.

9. Canule selon la revendication 8, **caractérisée en ce que** l'orientation privilégiée de la forme des perforations (8) s'étend dans la direction longitudinale du corps tubulaire (2).

10. Canule selon les revendications précédentes, **caractérisée en ce que** les perforations (8) ont un diamètre de trou D3, qui augmente, de préférence de manière continue, de l'intérieur vers l'extérieur du corps tubulaire (2).

11. Canule selon les revendications précédentes, **caractérisée en ce que** les perforations (8) situées du côté extérieur du corps tubulaire (2) sont arrondies.

12. Canule selon les revendications précédentes, **caractérisée en ce que** des marqueurs d'agent contrastant (9, 10) sont prévus au niveau de la zone d'extrémité avant (3) du corps tubulaire (2).

13. Canule selon la revendication 12, **caractérisée en ce que** les marqueurs d'agent contrastant (9, 10) définissent la zone des perforations (8) et/ou la zone de la courbure.

14. Canule selon la revendication 12 ou 13, **caractérisée en ce qu'**un marqueur d'agent contrastant (9) est positionné au début de la zone des perforations (8) et un marqueur d'agent contrastant (10) est positionné à la fin de la zone des perforations (8) ou à l'extrémité du corps tubulaire (2).

15. Canule selon les revendications précédentes, **caractérisée en ce que** la zone d'extrémité avant (3) de la canule (22) comporte à son extrémité distale une portion (23) au moins sensiblement rectiligne dans laquelle les perforations (8) sont situées de préférence exclusivement.

16. Canule selon les revendications précédentes, **caractérisée en ce que** l'auxiliaire d'insertion (7) comporte un mandrin (11) qui peut être poussé jusque dans le corps tubulaire (2) et/ou à travers celui-ci.

17. Canule selon les revendications précédentes, **caractérisée en ce que** l'auxiliaire d'insertion (7) comprend un fil métallique (12).

18. Canule selon la revendication 16 ou 17, **caractérisée en ce que** le fil métallique (12) est guidé dans ou sur le mandrin (11).
